(19) Europäisches Patentamt
European Patent Office
Office européen des brevets

(11) **EP 1 803 472 B1**

(12) **EUROPEAN PATENT SPECIFICATION**

(45) Date of publication and mention
of the grant of the patent:
**19.10.2016 Bulletin 2016/42**

(51) Int Cl.:
***A61L 17/14*** *(2006.01)*  ***A61K 35/36*** *(2006.01)*
***A61L 27/38*** *(2006.01)*

(21) Application number: **05790746.1**

(22) Date of filing: **17.08.2005**

(86) International application number:
**PCT/ES2005/000468**

(87) International publication number:
**WO 2006/035083 (06.04.2006 Gazette 2006/14)**

(54) **BIOMATERIAL FOR SUTURE**

**BIOMATERIAL FÜR NAHTMATERIAL**

**BIOMATERIAU POUR SUTURE**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **25.08.2004 ES 200402083**

(43) Date of publication of application:
**04.07.2007 Bulletin 2007/27**

(73) Proprietors:
• **Cellerix, S.A.**
**28760 Tres Cantos - Madrid (ES)**
• **Universidad Autónoma de Madrid**
**28049 Madrid (ES)**

(72) Inventors:
• **GONZÁLEZ DE LA PEÑA, Manuel**
**28760 Tres Cantos - Madrid (ES)**
• **FERNÁNDEZ MIGUEL, Gemma**
**28760 Tres Cantos - Madrid (ES)**
• **GARCÍA-OLMO, Damián,**
**Ciudad Univ. de Cantoblanco**
**28049 Madrid (ES)**

(74) Representative: **ABG Patentes, S.L.**
**Avenida de Burgos, 16D**
**Edificio Euromor**
**28036 Madrid (ES)**

(56) References cited:
**WO-A-02/067867    WO-A2-2005/062857
US-A- 5 855 619    US-B1- 6 174 333**

• **GIMBLE J M: "ADIPOSE TISSUE-DERIVED
THERAPEUTICS" EXPERT OPINION ON
BIOLOGICAL THERAPY, ASHLEY, LONDON, GB,
vol. 3, no. 5, August 2003 (2003-08), pages
705-713, XP009044158 ISSN: 1471-2598**
• **GIMBLE J M ET AL: "DIFFERENTIATION
POTENTIAL OF ADIPOSE DERIVED ADULT STEM
(ADAS) CELLS" CURRENT TOPICS IN
DEVELOPMENTAL BIOLOGY, ACADEMIC
PRESS, NEW YORK, NY,, US, vol. 58, 2003, pages
137-160, XP009034649 ISSN: 0070-2153**
• **AWAD H A ET AL: "Chondrogenic differentiation
of adipose-derived adult stem cells in agarose,
alginate, and gelatin scaffolds" BIOMATERIALS,
ELSEVIER SCIENCE PUBLISHERS BV.,
BARKING, GB, vol. 25, no. 16, July 2004 (2004-07),
pages 3211-3222, XP004490253 ISSN: 0142-9612**
• **YOUNG R G ET AL: "USE OF MESENCHYMAL
STEM CELLS IN A COLLAGEN MATRIX FOR
ACHILLES TENDON REPAIR" JOURNAL OF
ORTHOPAEDIC RESEARCH, THE JOURNAL OF
BONE AND JOINT SURGERY, INC.,, US, vol. 16,
no. 4, July 1998 (1998-07), pages 406-413,
XP008005059 ISSN: 0736-0266**
• **SHEN ZUN-LI ET AL: "A Schwann cell-seeded
intrinsic framework and its satisfactory
biocompatibility for a bioartificial nerve graft"
MICROSURGERY, vol. 21, no. 1, 2001, pages 6-11,
XP002526756 ISSN: 0738-1085**

- PASCUAL I ET AL: "Adipose-derived mesenchymal stem cells in biosutures do not improve healing of experimental colonic anastomoses." THE BRITISH JOURNAL OF SURGERY SEP 2008, vol. 95, no. 9, September 2008 (2008-09), pages 1180-1184, XP002526757 ISSN: 1365-2168
- CAPLAN ARNOLD I: "Mesenchymal stem cells: Cell-based reconstructive therapy in orthopedics" TISSUE ENGINEERING, vol. 11, no. 7-8, July 2005 (2005-07), pages 1198-1211, XP002526758 ISSN: 1076-3279
- GOMILLION ET AL: "Stem cells and adipose tissue engineering" BIOMATERIALS, ELSEVIER SCIENCE PUBLISHERS BV., BARKING, GB, vol. 27, no. 36, 1 December 2006 (2006-12-01), pages 6052-6063, XP005663847 ISSN: 0142-9612
- VAN EIJK F ET AL: 'Tissue Engineering of ligaments: A Comparison of Bone Marrow Stromal Cells, Anterior Cruciate Ligament, and Skin Fibroblasts as Cell Source.' TISSUE ENGINEERING. vol. 10, no. 5-6, May 2004, pages 893 - 903, XP008117512
- AWAD HANI A ET AL: 'Repair of patellar tendon injuries using a cell-collagen composite.' JOURNAL OF ORTHOPAEDIC RESEARCH. vol. 21, no. 3, 2003, pages 420 - 431, XP008117513
- KIM DONG-IK ET AL: 'Comparative study of seeding and culture methods to vascular smooth muscle cells on biodegradable scaffold.' JOURNAL OF MICROBIOLOGY AND BIOTECHNOLOGY. vol. 14, no. 4, May 2004, pages 707 - 714, XP008117562
- WAGNER W ET AL: "Comparative characteristics of mesenchymal stem cells from human bone marrow, adipose tissue, and umbilical cord blood", EXPERIMENTAL HEMATOLOGY, ELSEVIER INC, US, vol. 33, no. 11, 1 November 2005 (2005-11-01), pages 1402-1416, XP027605292, ISSN: 0301-472X [retrieved on 2005-11-01]

Remarks:

The file contains technical information submitted after the application was filed and not included in this specification

**Description**

**FIELD OF THE INVENTION**

This invention refers to a material for suturing and its applications. More specifically, the invention refers to a material for suturing covered with cells that contributes in a biologically active way to the tissue repair process and therefore the healing of wounds.

**STATE OF THE ART**

Any open wound involves a risk of infection and a channel for air and organic fluids to escape, making its closure an urgent necessity. In superficial skin wounds that do not pierce the dermis, a primary spontaneous closure takes place when the edges of the wound come together, while in wounds where there is a clear separation of tissue only surgical action (suturing of the wound) can achieve this primary closure, also known as first intention cure.

Traditionally, the suture has been the classical method for bringing the edges of the wound together in order to achieve the rapid tissue healing. First intention curing by suturing consists of bringing the edges of the wound together by introducing a suturing thread in the tissues using a metal needle joined at one of its ends, passing the needle successively between both sides of the incision, thereby passively facilitating the closure of the wound.

Sutures are also used in surgical practice to stop bleeding (haemostasis) and to repair organs and other structures of the human body. In some situations, these sutures are particularly delicate due to the healing difficulties of the tissues they are used on. This is the case of sutures used for the colon wall, for tendons as well as in microsurgery in nerve tissue and blood vessels.

One of the biggest disadvantages of tissue sutures is the fact that the diameter of the needle is larger than that of the thread, so that the point where the needle is inserted is not taken up completely by the thread, thus generating areas where fluids can escape. This deficient closure of the wound is frequently the cause of post-operative complications, such as in the case of intestinal anastomosis in patients with carcinoma or diverticulosis who have had part of their intestine removed, after which the two healthy ends are joined together. In said patients, when the closure is incomplete, faecal matter can leak into the surrounding tissues, which is a cause of peritonitis and places the patient's life at risk. This risk is higher in patients with thinner intestinal walls, as in the case of patients with intestinal inflammatory disease. In an attempt to avoid such leaking passively, biomedical adhesives are used that will be applied to the suture points to seal the opening caused when the needle is passed through the tissue.

The use of staples is an alternative to the classical suturing method. It enables the primary closure of the tissue to be carried out in less time, reduces the loss of blood, diminishes contamination and preserves blood flow. A limiting factor in the use of staples as a method of first intention cure is the need to have access to the upper and lower part of the tissues being joined together. Furthermore, the force exerted when inserting the staples may cause the tissue to tear. One solution to this problem, in a new attempt at passive contention, i.e., without contributing in a biologically active way to improving the tissue healing and repairing process, is to apply bioadhesives to the area where the staples are inserted.

The biocompatible adhesives work by facilitating the apposition of the tissues, providing a stable and regular biomechanical tension force all along the incision, which helps to maintain tissue structure around the wound. They can be divided into two categories: biological adhesives, synthesised from plasmatic proteins and synthetic polymers, primarily cyanoacrylate and its derivatives.

The biggest disadvantage of biological adhesives is the risk of viral transmission. Moreover, there are several disadvantages to the synthetic bioadhesives currently available. Most of them come in liquid form and are therefore difficult to apply, so their use is restricted to superficial wounds. On the other hand, they are allergenic and potentially toxic substances. It has been described that these materials induce an inflammatory response in the body, which contributes to a delay in the regeneration and healing of the tissue, thus greatly limiting their usefulness (Aronson et al., 1970; Milde et al., 1989). Hence, a method which enables the wound to close and the suturing of tissues without inducing an inflammatory response, without the need to use this type of adhesives, would be a particularly important advance in the case of internal tissue suturing.

On the other hand, sutures have now evolved to the point where there are sutures specifically designed for each type of operation. The surgeon chooses the suture based on the nature of the procedure, the characteristics of the patient, the tension which the suture must support, ... There is a wide variety of suturing threads available on the market today: absorbable and non-absorbable, with monofilament and multifilament structures, as well as being of natural and synthetic origin.

In order to improve the functional characteristics of suturing threads, there are diverse patents (GB577047, GB1401842, GB1430554, RU2125469) in which the threads are impregnated with germicide substances to prevent the suture points from being contaminated.

European patent EP0652017 protects biomaterials, the definition of which would include suturing materials, which have been coated to prevent blood constituents from adhering to the thread and thus delaying coagulation. Said patent also describes the use of anticoagulants and substances with antiinflammatory properties as the biomaterial coating.

Patent US6264675 protects a suturing material comprising a suturing thread coated with an adhesive material the adhesive properties of which are activated when inserted into the tissue to be repaired, which is joined to a needle at one of its ends. The bioadhesive used in said invention solves one of the main problems associated with the suturing method, since it prevents the loss of fluids through the needle insertion points. However, one of the disadvantages is the allergenic nature and potential toxicity inherent to the nature itself of the bioadhesives.

In addition, it is important to remember that all suturing materials induce "per se" an immunological response in the body due to the recognition of foreign matter, which systematically delays the natural healing of the tissue. Therefore, to achieve concealing the foreign matter from the immunological system by means of cellular coating, preferably an autologous one, would be again an especially significant advance in the case of compromised sutures.

In spite of the degree of sophistication reached by the sutures, the suturing methods described above contribute passively to the apposition of the tissues without participating in a biologically active way in the tissue healing.

On the other hand, there are diverse cellular populations in adults which are known to be capable of contributing to connective tissue repair. Thus, for example, the stromal cells of bone marrow contain, among others, a population of cells known as mesenchymal stem cells (Friedenstein et al., 1976; Caplan et al., 1991; Pittenger et al., 1999). Studies conducted on these cells have demonstrated that they can be differentiated into different lineages of mesenchymal cells, such as adipocytes (Beresford et al., 1972), chondrocytes (Johnstone et al., 1998), myoblasts (Wakitani et al., 1995), and osteoblasts (Haynesworth et al., 1992). Furthermore, multipotent stem cells which can be easily isolated have been identified (Zuk et al., 2001) in adipose tissue, which like bone marrow is derived from the embryonic mesoderm and is composed of a heterogeneous cell population. These cells are similar, though not identical, to the mesenchymal stem cells from bone marrow (De Ugarte et al., 2003) and are also able to be differentiated into multiple mesenchymal cell lineages (chondrocytes, osteocytes, adipocytes, and myoblasts). Moreover, like the mesenchymal stem cells from bone marrow, they have neuron differentiating capacity (Zuk et al., 2002).

The ability to join biological tissues together has been one of the principal challenges of biomedical research. The ideal suture is that which is resistant and easy to handle, does not induce an inflammatory response of the tissue and does not foster infection. In other words, that suture which not only allows to close the wound but also contributes to full healing.

Young et al. (1998, The Journal of Bone and Joint Surgery 16:406-13) describes a suture with a collagen matrix comprising mesenchymal cells and its use of Achilles tendon repair. The suture is coated with a suspension of bone marrow-derived mesenchymal stem cells combined with collagen.

Gimble et al. (2003, Expert Opinion on Biological Therapy 3:705-13) discloses the use of adipose tissue-derived stem cells for different therapeutic applications, such as the use of scaffolds or matrices coated with these cells for bone repair or wound healing.

This invention refers to a suturing material that makes it possible to bring the ends of the tissue together, facilitating the first intention cure and, at the same time, accelerating the repair process, contributing in a biologically active way to tissue healing. The use of such material also causes less inflammation of the sutured tissue, which reduces the time required for the open wound to heal, minimizing the risk of infection and the loss of body fluids and consequently the number of surgical failures.

**DESCRIPTION OF THE INVENTION**

The biomaterial for suturing referred to in this document comprises a physiologically compatible suturing material, known as support material, which is coated with a cellular population with proliferative and/or differentiation capacity, characteristics which are necessary to participate in the regeneration of the sutured tissue. Thus, this biomaterial for suturing is not limited to bringing the two ends of the open wound together, but also contributes actively to its healing, accelerating the tissue repair process. Said innovation represents an important advantage, especially in the case of sutured internal organs and particularly for intestinal anastomosis as a result of resectioning the gastrointestinal tract or urogenital area.

In a first aspect, the invention relates to a biomaterial for suturing comprising:

a) absorbable or non-absorbable threads

b) an adipose derived adult multipotent stem cellular population with proliferative and/or differentiation capacity coating said threads.

One aspect of the document describes a biomaterial for suturing which is useful as a therapeutic agent in the treatment of wounds, both accidental and surgical, and in suturing tissues. This biomaterial comprises a suturing support material and a cellular population coating said support material characterised by its proliferative and/or differentiation capacity.

In a particular embodiment, the biomaterial for suturing used includes absorbable threads, non-absorbable threads. In another particular embodiment, the biomaterial for suturing comprises natural origin threads or synthetic origin threads. In another particular embodiment, the biomaterial comprises threads with monofilament structure or threads with multifilament structure (also called braids).

In another particular embodiment, the biomaterial for suturing used includes absorbable threads, non-absorbable threads, natural origin threads, synthetic origin threads, threads with monofilament structure and threads with multifilament structure or braids, joined to a suturing needle.

In a particular embodiment, the invention provides a biomaterial for suturing comprising a suturing thread joined to a needle at one of its ends as support material and the adult multipotent stem cells as coating cellular population.

In a more preferred particular embodiment, the biomaterial for suturing used is an absorbable synthetic thread with monofilament structure joined to a suturing needle.

A disclosure of the document comprises using cells with proliferative and/or differentiation capacity as coating cellular population of a support material for suturing.

A particular disclosure of the document comprises using stem cells as coating cellular population of the biomaterial for suturing.

A more preferred particular disclosure comprises using pluripotent stem cells, capable of differentiating into any kind of tissue, as coating cellular population of the biomaterial for suturing.

An even more preferred particular disclosure comprises using multipotent stem cells, capable of differentiating into several kinds of tissue, as coating cellular population of the biomaterial for suturing.

The adult multipotent stem cells capable of differentiating into different cell types used as coating population are isolated from human adipose tissue. In humans, the preferred source of adipose tissue is subdermal fatty tissue, liposuction being the preferred collection method.

A particular embodiment of the invention comprises using adipose derived adult multipotent stem cellular population of an autologous, allogenic or xenogenic origin, or combinations of these, as the cellular population for coating.

Another particular embodiment of the invention comprises the use of adipose derived adult multipotent allogenic stem cellular population as the cellular population for coating the suturing biomaterial of the invention.

A more preferred embodiment of the invention comprises the use of an allogenic adipose derived adult multipotent stem cellular population as cellular population for coating the suturing biomaterial of the invention. The non-immunogenic character of mesenchymal stem cells isolated from bone marrow, which are not recognised by alloreactive T cells in *in vitro* assays has been described by other authors (Tse et al., 2003). Said characteristic provides the mesenchymal stem cells from an allogenic donor with the property of being able to be used in any patient, independently of whether there is a major histocompatibility complex (MHC) incompatibility. Therefore the use of mesenchymal stem cells from an allogenic donor involves an alternative source to autologous mesenchymal stem cells for their use in therapy.

Preferably, the coating cellular population will be autologous cells. An especially preferred particular embodiment comprises the use of adult multipotent autologous stem cells isolated from lipoaspirate as cellular population for coating the biomaterial for suturing of the invention (Example 1). The advantage of using adult autologous stem cells is that they are immunocompatible by nature and therefore, do not cause inflammation problems or rejection. Furthermore, there are no legal or ethical impediments to using them. In a preferred embodiment of the invention, said biomaterial for suturing comprises and adipose derived adult multipotent autologous stem cellular population as coating cellular population, their use being restricted to the patient from whom the cells are taken.

A particular disclosure of the document comprises using stem cells which express at least one inherent characteristic of a specialised cell as coating cellular population of the biomaterial for suturing to which the invention refers.

A preferred disclosure of the document comprises using progenitor cells from a specialised cellular lineage and obtained from the patient's stem cells which express at least one of the inherent characteristics of the specialised progenitor cell as coating cellular population of the biomaterial for suturing to which the invention refers. Therefore, this prevents the generation of inflammatory problems and rejection and the components of the suturing material would be concealed from the immunological system by coating it with autologous stem cells, which would undoubtedly improve the tissue repair process.

A preferred particular disclosure comprises using stem cells that have been induced to be differentiated *in vitro* into cells that express at least one inherent characteristic of a specialised cell as coating cellular population of the biomaterial for suturing.

A more preferred particular disclosure comprises using multipotent stem cells which have been induced to be differentiated *in vitro* into cells that express at least one inherent characteristic of a specialised cell, as the cellular population for coating the biomaterial for suturing. This includes but is not limited to the following cell types: epithelial cells, endothelial cells, adipocytes, myocytes, chondrocytes, osteocytes, neurons, astrocytes, oligodentrocytes, hepatocytes and pancreatic cells.

A preferred particular embodiment of the invention comprises the biomaterial for suturing of the invention in which the adipose derived adult multipotent stem cellular population for coating of the support material comprises genetically modified cells.

A preferred particular embodiment comprises the biomaterial for suturing in which the adipose derived adult multipotent stem cellular population of the support material comprises modified cells with a gene encoding a protein involved in tissue repair, including but not limited to growth factors, morphogenetic factors, structural proteins and cytokines.

Another preferred embodiment of the invention comprises the biomaterial for suturing of the invention in which the adipose derived adult multipotent stem cellular population for coating is composed of a heterogeneous cellular population. A heterogeneous cellular population is defined as that comprising different types of cells or cells in different stages of differentiation or a combination of both.

Another aspect of the invention comprises the use of adult multipotent stem cell population derived from adipose tissue with proliferative and/or differentiating capacity as coating cellular population of a threads for suturing.

In a particular embodiment, said use is characterised in that the adipose derived adult multipotent stem cellular population is an autologous, allogeneic or xenogeneic cellular population or a combination of these.

Another disclosure of the invention provides a method for obtaining the biomaterial for suturing of the invention in which the cellular population for coating is joined to the support material, preferably by adhesion.

An aspect of the invention provides a method for preparing a biomaterial for suturing of the invention, said method comprises:

1. expanding the adipose derived adult multipotent stem cellular population;
2. immersion of the suturing material in an appropriate culturing medium for that cellular population;
3. inoculating a suspension of the pre-viously cultured cellular population onto the suturing material;
4. culturing the previous preparation under the proper conditions, which includes but is not limited to dish cultures and dynamic cultures in tubes;
5. isolating the suturing material with the proper cellular coating.

In a particular embodiment, the adipose derived adult multipotent stem cellular population is joined to the support material by adhesion.

The expansion and growth stages of the cellular population used for coating the support material of the biomaterial will be obvious to any person skilled in the art.

A more preferred particular embodiment of the invention provides a method for preparing a biomaterial for suturing of the invention in which the threads have been previously coated with a material, the purpose of which is to improve the adhesion of the coating cell population. Said coating material of the threads is selected from the group consisting of peptides, protein antigens, sugars and lipids. In another more preferred particular embodiment, said coating material is extracellular matrix proteins from eukaryote cells or antibodies.

Another preferred particular disclosure provides a method for obtaining the biomaterial for suturing comprising genetically modifying the cellular population of choice previously to expanding said cellular population.

Another aspect comprises the biomaterial for suturing of the invention for its use in therapy.

Another aspect comprises the biomaterial for suturing of the invention for its use for bringing tissue edges in suturing of accidental or surgical wounds together and for tissue repairing.

Another aspect comprises the use of the biomaterial for suturing of the invention in the manufacture of material useful for bringing tissue edges together in suturing of accidental or surgical wounds and for tissue repairing.

Another aspect comprises the biomaterial for suturing of the invention for its use in hemostasis, organ transplants, surgery of the gastrointestinal tract, surgery of the urogenital tract, surgery of the respiratory tract, eye surgery, vascular surgery, plastic and reconstructive surgery, surgery on muscle tissue, epithelial tissue, nerve tissues as well as the repair of tendons, osseous tissue and cartilaginous tissue.

A more preferred particular disclosure of the document comprises the use of the sutures coated with mesodermal preferably autologous stem cells in those cases where the local inflammatory reaction generated by the suturing material could be harmful to the results of the surgical procedure.

An even more preferred particular embodiment comprises the use of the biomaterial for suturing in bringing the edges of the tissue together in any surgical activities where an improvement of local healing capacity is desired.

Another aspect of the invention comprises the use of the biomaterial for suturing of the invention in the manufacture of material useful for bringing the edges of tissue together in intestinal anastomosis.

Another aspect of the invention comprises the use of the biomaterial for suturing of the invention in the manufacture of material useful for the attachment of prostheses in place such as cardiac valves or neurosurgical valves.

Another particular disclosure of the document comprises the use of any prosthetic material (or device) used in medicine which is implanted in the human body and which frequently leads to biocompatibility problems, such as implant valves and surgical prostheses, which has been coated with cells.

## DESCRIPTION OF THE FIGURES

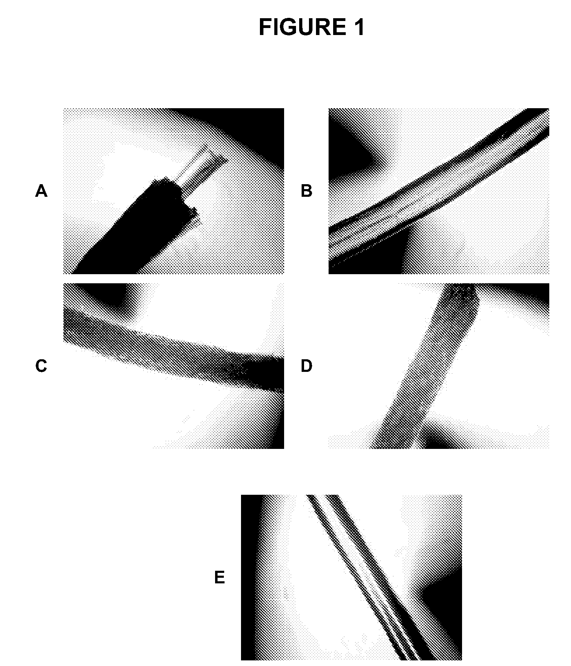

Figures 1a-1e show a phase contrast photomicrograph, in visible mode, of the different fragments of suturing thread used as support material in example 1. Figure 1 a shows the type of absorbable thread vicryl (Ethicon) ref. V460; figure 1b shows the type of absorbable thread monocryl (Ethicon) ref. Y3110; figure 1c shows the type of absorbable thread Dexon II (USS-DG) Ref. 9819-41; figure 1d shows the type of absorbable thread Safil quick (B/Braun) ref. 0046030 and figure 1e shows the type of non-absorbable thread Ethilon (Ethicon) ref. W1621.

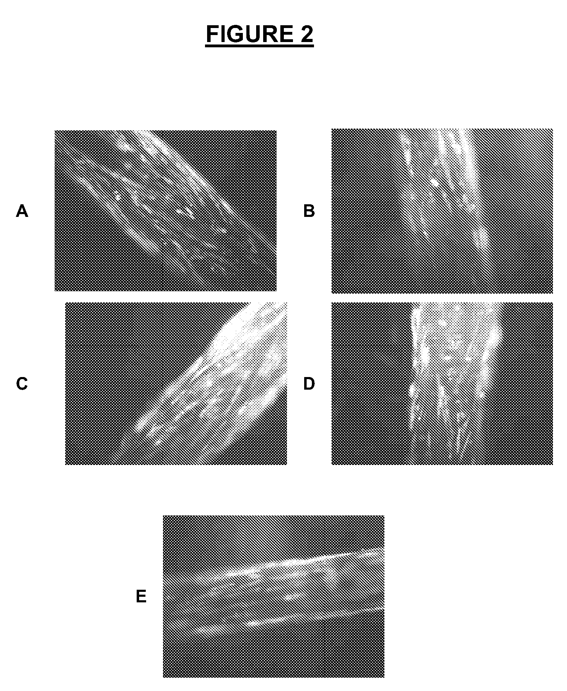

Figures 2a-2e show a phase contrast photomicrograph, in ultraviolet mode, the degree of cellular coating achieved in the fragments of suturing threads used in Example 1 after one week of incubation. Figure 2a shows the type of absorbable thread vicryl (Ethicon) ref. V460; figure 2b shows the type of absorbable thread monocryl (Ethicon) ref. Y3110; figure 2c shows the type of absorbable thread Dexon II (USS-DG) Ref. 9819-41; figure 2d shows the type of absorbable thread Safil quick (B/Braun) ref. 0046030 and figure 2e shows the type of non-absorbable thread Ethilon (Ethicon) ref. W1621.

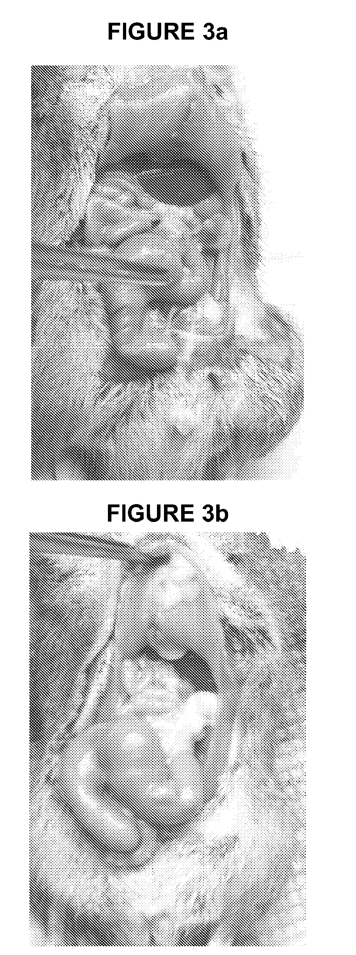

Figures 3a-3b show the general appearance of the abdominal cavity of rats after being laparotomised on the fourth post-operative day. A comparison of the general appearance (inflammation, general adhesions...) enables us to differentiate two patterns in the evolution of the anastomotic suture healing.

Figure 3a shows a photograph of one of the rats (no. 1) belonging to group A (surgery performed with the biomaterial for suturing of the invention). Figure 3b shows a photograph of one of the rats (no. 3) belonging to group B (surgery performed with Vicryl® 4/0 thread)

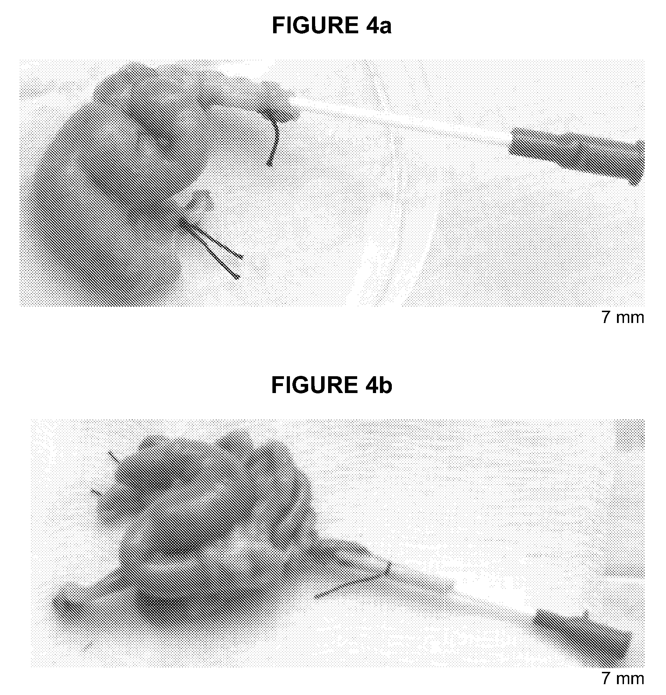

Figures 4a-4b show a photograph of a colic segment which contained the anastomosis once a catheter had been introduced at the proximal end, before determining the rupture pressure. Figure 4a shows a photograph of the colic segment of one of the rats (no. 1) belonging to group A (surgery performed using the biomaterial for suturing of the invention). Figure 4b shows a photograph of the colic segment of one of the rats (no. 3) in group B (surgery performed with Vicryl® 4/0 thread). For the purpose of understating the scaling of the images and to compare the degree of inflammation of the resected colic segments shown in figures 4a and 4b, the length of a portion of the catheter, the real size of which is 7 mm, has been used as a reference, and it has been indicated by a mark, used as reference scale, at the bottom of the figures.

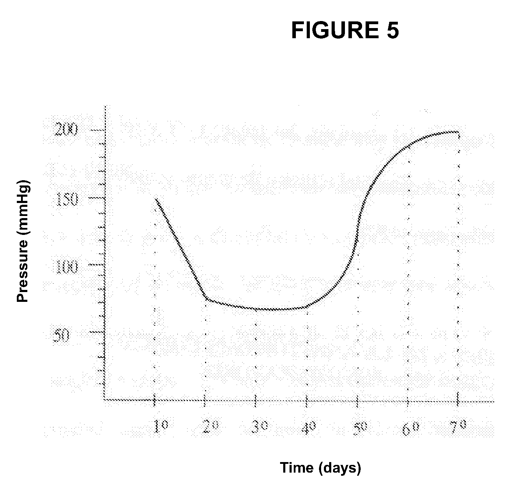

Figure 5 shows a diagram that illustrates the variation in the physical resistance of a standard colic suture in the presence of increased intraluminal pressures, depending on how much time has elapsed since it was carried out. Hence, it has been observed that within the first few hours after surgery there is a decline in the resistance, the lowest levels being detected between the third and fourth days after surgery.

## DETAILED DESCRIPTION OF THE EMBODIMENTS

The invention provides a biomaterial for suturing comprising:

a) absorbable or non-absorbable threads
b) an adipose derived adult multipotent stem cellular population with proliferative and/or differentiation capacity coating said threads.

The invention also provides a method for preparing a biomaterial for suturing of the invention, said method comprises:

1. expanding the adipose derived adult multipotent stem cellular population;
2. immersion of the suturing material in an appropriate culturing medium for that cellular population;
3. inoculating a suspension of the previously cultured cellular population onto the suturing material;
4. culturing the previous preparation under the proper conditions, which includes but is not limited to dish cultures and dynamic cultures in tubes;

5. isolating the suturing material with the proper cellular coating.

The invention also provides the use of the biomaterial for suturing of the invention for its use in therapy.

The invention also provides the biomaterial for suturing of the invention for its use in hemostasis, organ transplants, surgery of the gastrointestinal tract, surgery of the urogenital tract, surgery of the respiratory tract, eye surgery, vascular surgery, plastic and reconstructive surgery, surgery on muscle tissue, epithelial tissue, nerve tissues as well as the repair of tendons, osseous tissue and cartilaginous tissue.

The invention also provides the biomaterial for suturing of the invention for its use for bringing tissue edges in suturing of accidental or surgical wounds together and for tissue repairing.

The invention also provides the use of the biomaterial for suturing of the invention in the manufacture of material useful to for bringing tissue edges together in suturing of accidental or surgical wounds and for tissue repairing.

The invention also provides the use of the biomaterial for suturing of the invention in the manufacture of material useful for bringing the edges of tissue together in intestinal anastomosis.

The invention also provides the use of the biomaterial for suturing of the invention in the manufacture of material useful for the attachment of prostheses.

The invention also provides the use of adult multipotent stem cell population derived from adipose tissue with proliferative and/or differentiating capacity as coating cellular population of a threads for suturing.

The document describes a biomaterial for suturing comprising a support material for suturing, preferably a suturing thread, and a cellular population coating said support material. This coating cellular population is characterised by its proliferative and/or differentiation capacity.

In one particular disclosure, the document describes a biomaterial for suturing comprising a suturing thread joined to a needle at one of its ends as the support material and adult autologous stem cells derived from lipoaspirate as coating cellular population.

The following examples are given to illustrate but do not limit this invention.

### EXAMPLE 1: Suturing threads coated with adult human stem cells derived from adipose tissue

The object of this experiment was to study the capacity of a certain type of cell to adhere to different types of suturing threads acting as support material of the biomaterial for suturing of this invention.

### 1.1. Materials

Five different types of suturing thread, all of the same thickness 3-0 (2 Ph.Eur.) were used. All of them of synthetic origin (Table 1).

Table I

| No. Name | Type | Trademark | Reference |
|---|---|---|---|
| 1. VICRYL | Polyglactin 910. **Braiding.** Violet. **Absorbable** suture | ETHICON | V460 |
| 2. DEXON II | Polyglycolic Acid **Braiding.** Green. **Absorbable** suture | USS-DG | 9819-41 |
| 3. MONOCRYL | Polyglecaprone 25. **Monofilament.** Violet. **Absorbable** suture | ETHICON | Y3110 |
| 4. SAFILQUICK | Polyglycolic Acid **Braiding.** White. **Absorbable** suture | B-BRAUN | 0046030 |
| 5. ETHILON | Polyamide 6 **Monofilament.** Blue. **Non-absorbable** suture | ETHICON | W1621 |

Adherent adult human lipoaspirate-derived stem cells (LDSC) were used as coating cellular population, after first being transduced with retroviral vectors coding Cop-GFP, green fluorescent protein, used as gene marker.

### 1.1.1. Isolation of LDSC.

The adipose tissue was obtained by liposuction. A cannula with a blunt end was introduced into the subcutaneous space through a small periumbilical incision (less than 0.5 cm in diameter). The suction was performed by moving the cannula along the adipose tissue compartment located under the abdominal wall, thus aiding the mechanical disruption of the adipose tissue. To minimise the loss of blood, a saline and epinephrine solution was injected as vasoconstriction agent. 80-100 ml of raw lipoaspirate cells were obtained from each patient using this procedure.

The lipoaspirate was then washed with a phosphate buffer solution (PBS). The adipose tissue was then disrupted by digestion of the extracellular matrix with type II collagenase in saline solution (5 mg/ml) at 37° for 30 minutes to release the cellular fraction. The collagenase was inactivated by adding an equivalent volume of DMEM medium with 10% foetal bovine serum. Said cellular suspension was centrifuged at 250 g for 10 minutes to obtain a cell deposit. The cells were resuspended in DMEM medium with 10% foetal bovine serum. $NH_4Cl$ was added at a final concentration of 0.16M and incubated for 10 minutes at room temperature to induce the lysis of erythrocytes present. The suspension was centrifuged at 250-400 g and resuspended in DMEM-10% FBS with 1% ampicillin-streptomycin. Finally, the cells were plated at a rate of 20-30,000 cells per $cm^2$.

### 1.1.2. Culturing of LDSCs

The cells were kept in culture for 20-24 hours at 37° in an atmosphere with 5% $CO_2$. After 24 hours of incubation, the plates were washed with PBS to eliminate the cells that had not adhered, as well as cellular remains.

### 1.1.3. Transfection of adherent LDSCs with Cop-GFP gene marker

Transfection of the LDSCs which showed adherence characteristics by means of transduction with supernatant containing retroviral particles, RetroFect®, pseudotyped with VSV-G (vesicular stomatitis virus glycoprotein) which code the Cop-GFP gene marker. Cop-GFP is a copepod green fluorescent protein (*pontellina plumata*) which makes it easy to identify and select the infected cells by direct fluorescence. The fluorescent protein does not enter the cellular nucleus. This is an advantage since the cells that are expressing the Cop-GFP protein can be easily distinguished from interfering fluorescent particles.

The method used in the transduction of the adherent LDSCs consisted of:

1. The polybrene was added (adjuvant to retroviral transduction) to the retroviral supernatant at a final concentration of 8 $\mu$g/ml of polybrene.

2. The medium of the target cells was changed and replaced by retroviral particles using approximately 106 $\mu$l of retroviral supernatant (including additives) per $cm^2$ of surface.

3. A dynamic transduction method was used to infect the cells. The cells were centrifuged at 1000 g for 60 minutes at 37°C in the presence of retroviral supernatant.

4. The supernatant was removed and a fresh culturing medium of complete DMEM was added (DMEM-10% FSB with 1% ampicillin-streptomycin). The transduced cells were cultured for 48-72 hours in said culturing medium.

5. The cells were removed from the culture plates with a mixture of trypsin-EDTA.

6. An aliquot was analysed by flow cytometry; the rest of the cells were transferred to another culturing container for expansion.

### 1.2. Adhesion assay of cells to the suturing threads

Five different types of suturing threads were assayed as support material. Fragments measuring approximately 1 cm long were cut, introducing 2 thread fragments per well, on 24-well culture plates. Figures 1a-1e show a phase contrast photomicrograph, in visible mode, of the different types of suturing threads used as support material

1. 0.5 ml of the complete DMEM culturing medium was added to each one of the wells to moisten the threads and to find out if the threads would stay submerged in the culturing medium or if they would float. The tested threads either did not float or when forced under the medium they remaining at the bottom of the wells.

2. A suspension of LDSC cells was prepared in a concentration of 50,000 cells/ml and 1 ml of this suspension was added to each well.

3. The culture plates onto which the threads were deposited were cultured in the presence of the cellular suspension in an atmosphere with 5% $CO_2$ at 37°C for 20-24 hours.

4. The degree of cellular coating of the threads achieved after 20-24 hours of incubation was observed under the microscope, in visible mode and fluorescent mode.

In visible mode, it is not possible to detect the cells on the threads but it did allow to detect the presence of very disperse cells at the bottom of the well. However, in fluorescent mode, thanks to the expression of the Cop-GFP protein in the transfected LDSC cells, some individual fluorescent cells were observed on the threads, although very few in number.

5. The degree of cellular coating of the threads achieved after 48 hours of incubation was observed under the microscope in visible mode and fluorescent mode. No significant changes were observed in the coating of the threads compared to 24 hours of incubation. The threads were moved to plates with a new culturing medium. Starting at this time, the culturing medium was changed every 2-3 days according to the calendar.

6. The degree of cellular coating of the threads achieved after 72 hours of incubation was observed under the microscope in visible mode and fluorescent mode. Groups of cells on some of the tested threads could be observed and photographed, it was even possible to differentiate individual cells by distinguishing the nucleus of said cells, since Cop-GFP is not expressed in the cell's nucleus.

7. After one week of incubating the transduced LDSC cells with the Cop-GFP protein in the presence of the threads, an increase in the degree of cellular coating of the surface of the threads was observed, thanks to the cells generated by the division of the cells which initially were adhered to the threads. Figures 2a-2e show by means of a phase contrast photomicrograph, in ultraviolet mode, the degree of cellular coating achieved in the fragments of suturing threads used, after one week of incubation.

### Example 2: Use of the biomaterial for suturing for intestinal anastomosis.

The object of this assay has been to determine the characteristics of the biomaterial for suturing provided by this invention and the advantages that its use offers compared to conventional suture threads by performing colical anastomosis in rats.

After performing the anastomosis with the biomaterial for suturing of the invention and simultaneously with non-cell-coated threads used as a negative control, a series of parameters was determined which allow to evaluate the status of the anastomotic lesion and compare the results obtained with both types of thread.

#### 2.1. Surgical Procedure

#### 2.1.1. Animals and suturing material

12 adult BDIX rats with weights ranging between 130-260 grams were used in the experiment. Two of the specimens were used to obtain rat stem cells from the subdermal adipose tissue and 10 were used to study the colic sutures. The cell-coated threads were prepared following a protocol similar to that illustrated in Example 1. The BDIX rats are syngenic which means that they are genetically identical and immunologically compatible. Each rat was identified by a number from 1 to 10 and each one was assigned a batch of sutures with the same number.

The sample was divided into two groups depending on the suturing material used for the anastomosis:

- Group A (5 rats): Simple colic anastomosis performed with the biomaterial for suturing of the invention. More specifically, Vicryl® (polyglactin 910) 4/0 threads were used, an absorbable braided suture, coated with cells derived from the adipose tissues of BDIX rats. The same protocol as described in Example 1 was used to prepare the biomaterial for suturing.
- Group B (5 rats): Considered as control group. Simple colic anastomosis performed with Vicryl® (polyglactin 910) 4/0 threads.

50% of the suture batches were cultured in the presence of stem cells (biomaterial for suturing of the invention) and the other half were incubated, in identical conditions, in the presence of the culturing medium only. Therefore, it was impossible to differentiate the two types of suturing used by their appearance. This was a blind study since the surgeons did not know which type of thread was used in each procedure.

#### 2.1.2. Simple colic anastomosis

The rats were laparotomised under general anaesthesia after 24 hours of fasting with "ad libitum" water. The colon was completely sectioned at the midpoint of the transversal colon, taking care not to damage the margin vascularization and to prevent haemorrhaging. An everted end-to-end anastomosis on a single plane was then performed with at least 6 stitches. Each stitch was tied three times. When the anastomosis was complete, the colon was put back into the abdominal cavity and the laparotomy was closed with 0 silk thread on two planes.

#### 2.2. Evaluation of healing

The animals were sacrificed by decapitation on the fourth post-operative day. With the animal in asystole, the abdomen was opened and dehiscence, dilation, obstruction, general adhesions, difficulty in separating general adhesions and determination of adhered structures were evaluated.

Evaluation criteria of the variables under study:

1. Clinical dehiscence (De): Existence of free colic content in the peritoneal cavity.

2. Dilation (Di): Considered positive when the diameter of the pre-anastomotic transversal colon was at least two times larger than the diameter of the post-anastomotic transversal colon.

3. Obstruction (Ob): Absence of faecal content in the distal colon to the anastomosis.

4. General adhesions (GA): A measurement based on a qualitative evaluation scale of the number of adhesions in the peritoneal cavity was performed (Ellis H., 1962; Verreet PR et al, 1992). Four levels or degrees were established: 0=no adhesions; 1 = very localised adhesions; 2 = local-regional adhesions; 3 = diffuse adhesions.

5. Separation of the general adhesions (Se). Lysis by traction: Three levels were established: 1 = easy, most of the adhesions could be separated with gentle traction; 2 = moderate, most of the adhesions could be separate with a blunt instrument; 3 = difficult, most of the adhesions required a sharp instrument to be separated.

6. Adhered structures (AS): Which anatomical structures had been adhered to the anastomotic circumference is evaluated. 4 categories were established: 1 = epiploon, 2= small intestine, 3=colon, 4=other locations.

The measurement of resistance to intraluminal pressure can be expressed as the rupture pressure, i.e., the pressure at which an anastomosis subject to growing intraluminal pressure is disrupted or as the rupture tension which expresses the circular tension to which the wall is subjected at the time of the rupture.

2.2.1. Measurement of rupture pressure (RP)

Using an infusion pump connected to a pressure-measuring system it is possible to determine the pressure at which a leak can occur through the anastomotic line (rupture pressure).
After drying a colic segment containing the anastomosis, the distal end was closed with a 1/0 silk suture. The proximal end was similarly closed after introducing an intravenous perfusion catheter. Figures 4a-4b show a photograph of a colic segment which contained the anastomosis once a catheter had been introduced at the proximal end. The catheter is connected to a three-stage valve or "T" system in which one of the lines goes to the capsule of a pressure transducer that registers the pressure variations and sends the signals to a digital polygraph system. The data are then sent to a computer for analysis and storage.
The other line is connect to a perfusion pump filled with physiological solution tinted with methylene blue to observe the time and place of the rupture.

2.2.2. Rupture tension of the wall (RT)

The rupture tension reckoning is determined by measuring the anastomotic circumference after setting the piece in 20% formol for four days. A lengthwise cut was made with a scalpel in the colon segment containing the anastomosis, and with a graduated rule, the internal circumference (cn) of the piece was measured in tenths of a millimetre. Knowing the length of the internal circumference allows us to calculate the internal colon radius (r) applying the following formula: cn=2nr.
The rupture tension (RT) is a function of the rupture pressure (RP) and the internal colon radius (r) according to the Laplace Law. It is obtained by applying the following formula:

$$RT = 1.33 \times 10^3 \times RP \times r \quad (RT = \text{dinas/cm; } RP = \text{mmHg; } r=\text{cm})$$

2.3. Results

Colic anastomosis was performed on 10 adult BDIX rats distributed into two groups, A and B, according to the suturing material used. In order to evaluate the healing of the anastomosis, two of the animals operated on, one from each group, had to be excluded from the study. During the surgery, one of the animals suffered a tearing of the mesosigmoid while in another animal the anastomosis was torn when introducing the catheter in the distal end of the colic at the moment of measuring the rupture pressure.
Table II shows the results obtained for the different variables analysed in the evaluation of the anastomotic suture.

**Table II**

| ANIMAL | De | INFLAMMATION | | ADHESIONS | | | RESISTANCE | | |
|---|---|---|---|---|---|---|---|---|---|
| | | Di | Ob | GA | Se | As | RP (mmHq) | r (cm) | RT x10$^3$(dinas/cm) |
| 1 | NO | NO | NO | 2 | 1 | 1,3 | 43.1 | 0.38 | 21.78 |
| 4 | NO | YES | NO | 2 | 1 | 1,2 | 41.73 | 0.35 | 19.43 |
| 6 | NO | YES | NO | 2 | 1 | 2,4 | 63.1 | 0.22 | 18.46 |
| 7[a] | NO | YES | NO | 3 | 3 | 1,2 | 36.7 | 0.27 | 13.18 |
| MEDIA | | | | | | | **46.16** | 0.31 | **19.03** |
| 2 | NO | YES | YES | 3 | 3 | 1,2,3,4 | 52 | 0.24 | 16.60 |
| 3 | NO | YES | NO | 2 | 3 | 1,2,3 | 50.47 | 0.35 | 23.49 |
| 5[b] | NO | NO | NO | 3 | 3 | 1,2,3 | 52.06 | 0.25 | 17.31 |
| 10 | NO | YES | YES | 3 | 3 | 1,2,4 | 24.4 | 0.24 | 7.79 |
| MEDIA | | | | | | | **44.73** | 0.27 | **16.06** |

**Group A:** 1,4,6,7          $T_{student}$          0.879          0.624
**Group B:** 2,3,5,10

COMMENTS:
blind inverted          b 7 stitches
ABBREVIATIONS:

Clinical dehiscence (De)          General adhesions (GA)          Rupture pressure (RP)

Dilation (Di)          Separation of general adhesions (Se)          Radius (r)

Obstruction (Ob)          Adhered structures (AS)          Rupture tension (RT)

2.4. Discussion

The healing of the anastomotic wound was evaluated on the fourth post-operative day, as this is considered the most critical day in the evolution of colic anastomosis (Ravo, 1988).

After opening the abdominal cavity, the quadrants were pulled back exposing the entire peritoneal cavity. Figures 3a-3b show the general aspect that the abdominal cavity of the rats presented after being laparotomized on the fourth post-operative day. Surprisingly, an assessment of the general appearance (inflammation, general adhesions,...) is sufficient to differentiate two different patterns in the evolution of the healing of the anastomotic suture. Thus, the animals on which conventional suturing threads had been used (Group B) show a higher level of general inflammation and a higher number of structures adhered to the intestine than those animals on which the surgery was carried out using the biomaterial for suturing of the invention (Group A).

First of all, it was determined that the surgical handling of the biomaterial for suturing of the invention is identical to that of conventional threads. The presence of cells coating the threads does not alter the manageability of the sutures.

A series of variables, dehiscence, dilation, obstruction, general adhesions, separation of adhesions, adhered structures, rupture pressure and tension pressure, was determined in order to evaluate the healing of the anastomotic suture.

1. Dehiscence: No faecal matter was observed in the peritoneal cavity in either one of the groups. The surgical techniques as well as the suturing material used were appropriate for the type of rat used.

2. Dilation: Dilation of the pre-anastomotic colon was observed in 75% of the cases in both groups.

3. Obstruction: Although the dilation of the pre-anastomotic segment was similar in both groups, the clinical repercussion was different. Thus, 100% of the animals from the group treated surgically with the biomaterial of the invention (Group A) showed no intestinal obstruction, while obstruction was observed in 50% of the animals treated with conventional sutures (Group B). The absence of colic obstruction implies a series of clinical advantages in terms of the speed of the patient's recovery and the reduction of post-operative complications.

4. General Adhesions: A qualitative assessment of the number of adhesions on the peritoneal cavity reveals that in Group A 75% of the cases shows local-regional adhesions, while in Group B 75% of the cases shows diffuse adhesions.

5. Separation of adhesions: In Group A 75% of the cases belongs to Level 1, or adhesions which are easily separated. Only one case (25%) was classified as Level 3, requiring the use of a sharp instrument for its separation. Nevertheless, this animal showed as special feature that the cecum's position had been inverted during the surgical procedure. On the contrary, 100% of the cases from Group B belongs to Level 3.

6. Adhered structures: In group A, the epiploon is adhered in 75% of the cases and 100% of the cases show adhesions to two different structures. In Group B, 100% of the cases had the epiploon and small intestine adhered. In 75% of the cases adhesions to three different structures were observed and in the remaining case adhesions to four different structures were observed.

The adhesions have pathological importance since they alter the normal physiology of serous surfaces. Surprisingly, the animals belonging to Group A showed a more regional pattern of adhesions, easier to separate and a lower number of adhered structures, which implies a reduction of the complications caused by intraperitoneal adhesions: intestinal obstruction, chronic abdominal pain and infertility. The use of a foreign material in the abdominal cavity produces a high level of adhesions (Ellis H, 1962, Zarapico et al, 1972). The decrease in the adhesions observed in the assay could have been due to the fact that the biomaterial of the invention is not recognised as something foreign by the body.

The purposes of using a physical method for evaluating the healing of the anastomosis is to ensure that it is as comprehensive as possible, since the use of other methods, such as biochemical or histological, give us only a partial view of these processes at the anastomosis level. The physical resistance of a colic suture before the intraluminal pressure increases varies depending on the time which has elapsed since its performance. Hence it has been observed that after the first post-operative hours there is a drop in resistance, the lowest values being detected between the third and fourth post-operative days. From this moment on, a rapid increase in the resistance occurs, reaching values close to those of the resistance of the colic wall in its physiological state (figure 5) on the seventh post-operative day. This inflection in the curve of the anastomotic force around the fourth post-operative day is closely linked to the cellular and chemical processes that take place during the inflammatory and repair process.

In Group A, the average resistance of the anastomosis, calculated as the rupture pressure, is 46.1575 mmHg, which is higher than the average resistance observed in Group B: 47.7325 mmHg.

The disadvantage of measuring the resistance to intraluminal pressure by determining the rupture pressure is that it does not take the diameter of that colon segment into account. If we measure the rupture pressure without taking the diameter of the colon segment into account, we set the Laplace Law $(T=P \times r)$ aside and we do not consider the fact that for the same rupture pressure the tension which the wall supports is greatest in the area with the largest radius (Asencio F. et al, 1989). When calculating the average rupture tension, we checked that in Group A the intestinal wall supports an average rupture tension of $19.03 \times 10^3$ dinas/cm, which is higher than the average resistance found in Group B: $16.06 \times 10^3$ dinas/cm.

Therefore, although the differences as far as rupture pressure are not statistically significant, the calculation of the rupture tension corroborated the results obtained: the suture carried out by the biomaterial of the invention shows greater resistance to pressure than the suture carried out by conventional thread. Furthermore, the maximum rupture resistance value was belongs to one of the animals from Group A and the lowest value was observed in Group B. A higher resistance to rupture of the anastomotic suture implies a lower risk of dehiscence, separation of part of the anastomosis, which is a serious complication and one of the leading causes of post-operative death in colic surgery.

### 2.5. Conclusion

An evaluation of the general appearance (inflammation, general adhesions,..) is sufficient to differentiate two patterns in the healing evolution of the anastomotic suture. The animals on which conventional suturing threads were used (Group B) presented a higher degree of general inflammation and a higher level of adhesions (more diffuse adhesions, a greater number of adhered structures and a higher degree of difficulty in their separation) than those animals in which the surgery was performed using the biomaterial for suturing of the invention (Group A). The use of the material for suturing of the invention induces a lower inflammatory response giving rise to a reduction of the typical post-anastomotic complications.

Likewise, in those animals that have been operated on with the biomaterial for suturing of the invention (Group A) higher resistance to increasing intraluminal pressure of the colic sutures and therefore a lower risk of dehiscence is observed.

On the fourth post-operative day, considered as the most critical day in the evolution of colic anastomosis, we could conclude that the anastomotic sutures performed with the biomaterial for suturing of the invention show better evolution with regard to those that have been performed with conventional suture threads. The biomaterial for suturing of the invention is not limited to bringing tissue edges together, but it also contributes in a biologically active way to the tissue healing by accelerating the repair process.

### EXAMPLE 3: Use of the biomaterial for suturing in intestinal anastomosis. Evaluation of mid-term healing

Once the features of the biomaterial for suturing provided by the present invention and the advantages of its use over conventional suturing threads were studied in the embodiment of short-term colic anastomosis in rats (fourth post-operative day), a second assay was performed for the purpose of determining if the two patterns in terms of inflammation and adhesions observed on the fourth post- anastomosis day are maintained.

The evolution of the anastomotic wound was studied in animals sacrificed one and two weeks after the surgical procedure in order to evaluate the mid-term healing evolution. As in Example 2, after the anastomosis was performed with the biomaterial for suturing of the invention and at the same time with conventional suturing threads, used as a negative control, a series of parameters were determined that allowed to evaluate the state of the anastomotic lesion and to compare the results obtained in the two study groups.

#### 3.1. Surgical Procedure

20 adult BDIX male rats were used in the experiment, each weighing between 130-260 grams. Two assays were conducted and the animals were sacrificed in the first and second post- anastomosis weeks. Each one of the rats was identified in each assay with a number from 1-10, pairing the animal with a suture batch with the same number.

The animals used were divided into two groups according to the material for suturing used in the anastomosis:

- Group A (5 rats): Simple colic anastomosis performed with the biomaterial for suturing of the invention. More specifically, Vicryl® (polyglactin 910) 4/0 threads were used, an absorbable braided suture, which had been coated with cells derived from the adipose tissues of BDIX rats. The same protocol as described in Example 1 was used to prepare the biomaterial for suturing.
- Group B (5 rats): Considered the control group. Simple colic anastomosis performed with Vicryl® (polyglactin 910) 4/0 threads.

As in Example 2, 50% of the suture batches were cultured in the presence of stem cells (biomaterial for suturing of the invention) and the other half were incubated, in identical conditions, in the presence of the culturing medium only, being impossible to differentiate the two types of suturing used by their appearance. Furthermore, the rats were operated on performing an intestinal anastomosis, as described in Example 2.

#### 3.2. Evaluation of healing

The healing of the anastomotic wound in the animals sacrificed one and two weeks after surgery was evaluated in order to follow-up on the mid-term evolution of healing. The variables studied are defined in Example 2.

#### 3.3. Results

A colic anastomosis was performed in 20 adult BDIX rats distributed into two groups of individuals which were analyzed at different times after surgery. At the same time, each group was divided into two subgroups A and B, according to the material for suturing used.

Table IIIa shows the results obtained for the different variables analyzed in the evaluation of anastomotic suture one week after the surgical procedure.

**Table IIIa**

| ANIMAL | De | INFLAMMATION | | ADHESIONS | | | RESISTANCE | | |
| | | Di | Ob | GA | Se | AS | RP (mmHg) | r (cm) | RT x$10^3$(dinas/cm) |
|---|---|---|---|---|---|---|---|---|---|
| 2 | NO | NO | NO | 2 | 2 | 1,2 | 124 | 0.366 | 60.36 |
| 3 | NO | NO | NO | 2 | 2 | 1,2 | 126.4 | 0.302 | 50.77 |
| 6 | NO | NO | NO | 2 | 2 | 1,2 | 138.54 | 0.398 | 73.33 |
| 7 | NO | NO | NO | 2 | 3 | 1,2 | 120.73 | 0.318 | 51.06 |
| 10 | NO | NO | NO | 2 | 2 | 1,2 | 108 | 0.366 | 52.57 |
| AVERAGE | | | | | | | **123.53** | 0.35 | **57.62** |
| 1 | NO | NO | NO | 3 | 3 | 1,2,4 | 119.7 | 0.429 | 68.08 |
| 4 | NO | NO | NO | 3 | 3 | 1,2 | 121.82 | 0.27 | 43.74 |
| 5 | NO | NO | NO | 2 | 3 | 1,2,3 | 92.78 | 0.35 | 43.16 |
| 8 | NO | NO | NO | 3 | 3 | 1,2 | 124 | 0.398 | 65.63 |
| 9 | NO | NO | NO | 3 | 3 | 1,2,3,4 | 119.68 | 0.35 | 55.71 |
| AVERAGE | | | | | | | **115.60** | 0.359 | **55.26** |
| **Group A:** 2,3,6,7,10 $\quad$ T$_{student}$ $\quad$ 0.325 0.738 $\quad$ **Group B:**1,4,5,8,9 | | | | | | | | | |
| ABBREVIATIONS: Clinical dehiscence (De) $\quad$ General adhesions (GA) $\quad$ rupture pressure (RP) Dilation (Di) $\quad$ Separation of general adhesions (Se) $\quad$ Radius (r) Obstruction (Ob) $\quad$ Adhered structures (AS) $\quad$ Rupture tension (RT) | | | | | | | | | |

Table IIIb shows the results obtained for the different variables analyzed in the evaluation of the anastomotic suture, two weeks after the surgical procedure.

**Table IIIb**

| ANIMAL | De | INFLAMMATION | | ADHESIONS | | | RESISTANCE | | |
| | | Di | Ob | GA | Se | AS | RP (mmHg) | r (cm) | TRx$10^3$(dinas/cm) |
|---|---|---|---|---|---|---|---|---|---|
| 1 | NO | NO | NO | 1 | 2 | 1 | 114.57 | 0.286 | 43.58 |
| 4 | NO | YES | NO | 2 | 3 | 1,2 | 80.2 | 0.366 | 39.04 |
| 6 | NO | YES | NO | 1 | 3 | 1 | 87.62 | 0.222 | 25.97 |
| 7 | NO | NO | NO | 1 | 3 | 1 | 105.6 | 0.302 | 42.41 |
| 9 | NO | YES | NO | 2 | 3 | 1,2 | 94.21 | 0.366 | 45.86 |
| AVERAGE | | | | | | | **96.44** | 0.308 | **39.37** |
| 2 | NO | YES | NO | 2 | 3 | 1,2 | 101.21 | 0.191 | 25.71 |
| 3 | NO | YES | NO | 2 | 3 | 1,2 | 87.62 | 0.127 | 14.8 |
| 5 | NO | NO | NO | 1 | 2 | 1 | 101.36 | 0.318 | 42.87 |
| 8 | NO | NO | NO | 2 | 3 | 1,2 | 110.63 | 0.318 | 46.79 |
| 10 | NO | YES | NO | 3 | 3 | | 107.92 | 0.27 | 38.75 |
| AVERAGE | | | | | | | **101.75** | 0.245 | **33.78** |

| **Group A:** 1,4,6,7,9<br>**Group B:** 2,3,5,8,10 | $T_{student}$ | 0.490 | 0.441 |

ABBREVIATIONS:

| Clinical dehiscence (De) | General adhesions (GA) | rupture pressure (RP) |
| Dilation (Di) | Separation of general adhesions (Se) | Radius (r) |
| Obstruction (Ob) | Adhered structures (AS) | Rupture tension (RT) |

### 3.4. Discussion

For the purpose of knowing if there are differences in the mid-term evolution of healing between the two study groups, healing of the anastomotic wound was evaluated one and two weeks after the surgical procedure. A series of variables, dehiscence, dilation, obstruction, general adhesions, separation of adhesions, adhered structures, rupture pressure and tension pressure, was determined for such evaluation.

### 3.4.1. Analysis the first post-anastomosis week:

1. Dehiscence: No faecal matter was observed in the peritoneal cavity in either one of the study groups. The observed results indicate that the surgical techniques as well as the suturing material used were appropriate.

2. Dilation: Dilation of the pre-anastomotic colon was not observed in either of the study groups.

3. Obstruction: The absence of dilation both in the group of animals treated surgically with the biomaterial for suturing of the invention (Group A) and in the group treated with conventional sutures (Group B) implies that there was no intestinal obstruction.

4. General Adhesions: A qualitative assessment of the number of adhesions on the peritoneal cavity revealed that in Group A 100% of the cases showed local-regional adhesions, while in Group B 80% of the cases showed diffuse adhesions.

5. Separation of adhesions: In Group A 80% of the cases was classified in Level 2 (moderate), the adhesions are separated using a blunt instrument. On the contrary, 100% of the cases from Group B was classified in Level 3, since it was necessary to use a sharp instrument to separate the adherences.

6. Adhered structures: In all the animals pertaining to group A, the epiploon and the small intestine (two different anatomical structures) were adhered. The Group B animals, despite obtaining heterogeneous results, generally presented a higher number of adhered structures: 100% of the animals had the epiploon and small intestine adhered, in 40% of the cases adhesions to three different structures were observed and one of the animals (20%) presented adhesions to four different structures.

7. Rupture pressure: The average resistance of the anastomosis in Group A, calculated as rupture pressure, is 123.53 mmHg, exceeding the average rupture pressure found in Group B: 115.60 mmHg.

8. Rupture tension: Rupture tension expresses the circular tension that the intestinal wall supports in the moment of rupture. In Group A the intestinal wall supports an average rupture tension of $57.62 \times 10^3$ dinas/cm, exceeding the average rupture tension of Group B: $55.26 \times 10^3$ dinas/cm.

There are generally no differences between the two study groups in terms of the evaluation of the inflammation. However, the pattern observed on the fourth post-anastomosis day in terms of adhesion (general adhesions, separation of adhesions, adhered structures) was maintained. Therefore, it was observed that the animals belonging to Group A, treated with the biomaterial for suturing of the invention, presented more localized and easier to separate adhesions and with a lower number of adhered structures.

As regards the resistance of the anastomotic line, even though there are no statistically significant differences, greater resistance to an increasing intraluminal pressure is maintained, both in relation to rupture tension as well as rupture pressure in those sutures performed with the biomaterial of the invention. Furthermore, the maximum rupture resistance value was seen in one of the animals belonging to the Group A and the lowest value was observed in the Group B, as occurred in the assay performed on the fourth post-anastomosis day.

3.4.2. Analysis the 2nd post- anastomosis week:

1. Dehiscence: No free colic matter was observed in the peritoneal cavity in 100% of the animals of both groups. The observed results indicate that the surgical technique as well as the suturing material used were appropriate.

2. Dilation: 60% of the animals in both groups presented dilation of the pre-anastomotic colon. No differences were observed in relation to the material for suturing used.

3. Obstruction: 100% of the intestinal obstruction-free results were obtained in both the group of animals treated surgically with the biomaterial for suturing of the invention (Group A) as in the group treated with conventional sutures (Group B).

4. General Adhesions: A qualitative assessment of the number of adhesions on the peritoneal cavity revealed that in Group A 60% were very localised and the remaining 40% were showed local-regionals. In Group B 60% were local-regional, 20% very localised and the remaining 20% diffuse adhesions.

5. Separation of adhesions: The intensity of adhesion to other organs has been high both in the animals treated with the biomaterial for suturing of the invention and in the animals treated with conventional sutures, in both groups 80% of the cases pertained to separation Level 3 (difficult), being necessary to use a sharp instrument to separate the adherences.

6. Adhered structures: 100% of the animals pertaining to group A presented epiploon adhesions, 40% of the cases further showing adhesions in the small intestine. In Group B, 100% of the cases presented adhesions in the epiploon. Furthermore, 80% of the cases presented adhesions to two different structures, 60% of them in the small intestine.

7. Rupture pressure: The average resistance of the anastomosis in Group A, calculated as rupture pressure, is 96.44 mmHg, less than the average rupture pressure found in Group B: 101.75 mmHg.

8. Rupture tension: In Group A the intestinal wall supports an average rupture tension of $39.37 \times 10^3$ dinas/cm, exceeding the average rupture tension of Group B: $33.78 \times 10^3$ dinas/cm.

There are generally no differences between the two study groups in relation to the evaluation of inflammation. In relation to the level of adhesions observed, the use of the biomaterial for suturing of the invention gives rise to more localised adhesions, with a lower number of adhered anatomical structures and with a similar degree of difficulty in their separation in relation to the use of conventional sutures.

In relation to the resistance of the anastomotic suture, when the rupture tension is analyzed we observed that the circular tension the intestinal wall is subjected to at the moment of the rupture is greater in the group of animals that had been operated on using the biomaterial for suturing of the invention (Group A).

Better evolution of the anastomotic wound is maintained in those animals that had been operated on using the biomaterial for suturing of the invention. Nevertheless, both in terms of the inflammation pattern and in terms of the adhesion pattern, the differences observed in the animals sacrificed two weeks after the surgery are milder than those observed on the fourth post-operative day and the first post-anastomosis week.

3.5. Conclusion

When the evolution of healing is evaluated on the fourth post- anastomosis day and on later weeks, we found that the biomaterial for suturing of the invention presents considerable advantages (lower degree of general inflammation, lower level of adherences and a greater resistance to intraluminal pressure) in comparison with the standard sutures. The biomaterial for suturing of the invention contributes in a biologically active manner in anastomotic wound healing, the differences in relation to the use of conventional sutures being more evident on the fourth post-operative day, considered critical in the evolution of the anastomotic wound. Said advantages are maintained in the mid-term despite being less significant.

## References

- Aronson SB, McMaster RP, Moore TE Jr, Coon MA. Toxicity of the cyanoacrylates. Arch Opthalmol. 1970 Sep; 8(3):342-9.
- Asencio Arana F, Martínez Soriano F, Fenollosa Vazquez R. Aproximación a los estudios de los anastomosis intestinales experimentales. Métodos bioquímicos, físicos y microangiográficos. Cir Esp 1989; 46:805-810.
- Beresford JN, Bennett JH, Devlin C, Leboy PS, Owen ME. Evidence for an inverse relationship between the differentiation of adipocytic and osteogenic cells in rat marrow stromal cell cultures. J. Cell Sci. 1992 Jun; 102(Pt 2): 341-51.
- Caplan AI. Mesenchymal stem cells. J Orthop Res. 1991 Sep; 9(5):641-50.
- De Ugarte DA, Morizono K, Elbarbary A, Alfonso Z, Zuk PA, Zhu M, Dragoo JL, Ashjian P, Thomas B, Benhaim P, Chen I, Fraser J, Hedrick MH. Comparison of multi-lineage cells from human adipose tissue and bone marrow. Cells Tissues Organs. 2003; 174(3):101-9.
- Ellis, H. the aetiology of postoperative abdominal adhesions. An experimental study. Br J Surg 1962; 50:10-16.
- Friedenstein AJ. Precursor cells of mechanocytes. Int Rev Cytol. 1976; 47:327-59.
- Haynesworth SE, Goshima J, Golberg VM, Caplan AI. Characterization of cells with osteogenic potential from human marrow. Bone. 1992; 13(1):81-8.
- Johnstone B, Hering TM, Caplan AI, Goldberg VM, Yoo JU. In vitro chondrogenesis of bone marrow-derived mesenchymal progenitor cells. Exp Cell Res. 1998 Jan 10; 238(1):265-72.
- Milde LN. An anaphylactic reaction to fibrin glue. Anesth Analg. 1989 Nov; 69(5):684-6.
- Pittenger MF, Mackay AM, Beck SC, Jaiswal RK, Douglas R, Mosca JD, Moorman MA, Simonette DW, Craig S, Marshak DR. Multilineage potential of adult human mesenchymal stem cells. Science. 1999 Apr2; 284(5411):143-7.
- Ravo B: Colorectal anastomotic healing and introcolonoic bypass procedure. Surg Clin North Am 1988; 68:1267-1294.
- Tse WT, Pendleton JD, Beyer WM, Egalka MC, Guinan EC. Suppression of allogeneic T-cell proliferation by human marrow stromal cells: implications in transplantation. Transplantation. 2003 Feb 15; 75(3):389-97.
- Verreet RP, Fakir C, Ohmann C, Roer HD. Preventing recurrent postoperative adhesions: An experimental study in rats. Eur Sug Res 1992; 21:267-273.
- Wakitani S, Saito T, Caplan AI. Myogenic cells derived from rat bone marrow mesenchymal stem cells exposed to 5-azacytidine. Muscle Nerve. 1995 Dec; 18(12):1417-26.
- Zarapico Romero M, Saez López de Rueda F. La asociación fibrinodesoxirribonucleasa en la profilaxis de la adhesiones peritoneales post-operativas. Rev Fac Med Sevilla 1972; 20:347-362.
- Zuk PA, Zhu M, Ashjian P, De Ugarte DA, Huang JI, Mizuno H, Alfonso ZC, Fraser JK, Benhaim P, Hedrick MH. Human adipose tissue is a source of multipotent stem cells. Mol Biol Cell. 2002 Dec; 13(12):4279-95.
- Zuk PA, Zhu M, Mizuno H, Huang J, Futrell JW, Katz AJ, Benhaim P, Lorenz HP, Hedrick MH. Multilineage cells from human adipose tissue: implications for cell-based therapies. Tissue Eng. 2001 Apr; 7(2):211-28.

## Claims

1. Biomaterial for suturing comprising:

   a) absorbable or non-absorbable threads
   b) an adipose derived adult multipotent stem cellular population with proliferative and/or differentiation capacity coating said threads.

2. Biomaterial, according to claim 1, wherein the threads are of natural or synthetic origin.

3. Biomaterial, according to claims 1-2, wherein the threads have a monofilament or multifilament structure.

4. Biomaterial, according to any one of the previous claims, wherein the thread is a suturing thread joined to a needle.

5. Biomaterial, according to any one of the previous claims, wherein the adipose derived adult multipotent stem cellular population of the support material is a heterogeneous cellular population.

6. Biomaterial, according to any one of the previous claims, wherein the adipose derived adult multipotent stem cellular population of the coating for the support material comprises genetically modified cells.

7. Biomaterial, according to claim 6, wherein the adipose derived adult multipotent stem cellular population of the support material comprises modified cells with a gene encoding a protein involved in tissue repair.

8. Biomaterial, according to any one of the previous claims, wherein the adipose derived adult multipotent stem cellular population for the support material comprises an autologous, allogeneic or xenogeneic cellular population or a combination of these.

9. Biomaterial, according to claim 8, wherein the adipose derived adult multipotent stem cellular population of the support material comprises an autologous cellular population.

10. Biomaterial, according to claim 8, wherein the adipose derived adult multipotent stem cellular population of the support material comprises an allogeneic cellular population

11. Method for preparing a biomaterial, according to any of the previous claims, comprising

   a) Expanding the adipose derived adult multipotent stem cellular population
   b) Immersion of the suturing material in an appropriate culturing medium for that cellular population.
   c) Inoculating a suspension of the previously cultured cellular population onto the suturing material.
   d) Culturing the previous preparation under the proper conditions.
   e) Isolating the suturing material with the proper cellular coating.

12. Method for preparing a biomaterial for suturing according to the previous claim, wherein the adipose derived adult multipotent stem cellular population is joined to the support material by adhesion.

13. Method for preparing a biomaterial, according to claim 11, further comprising a previous coating of the threads that facilitates the adherence of the cellular population.

14. Method for preparing a biomaterial, according to claim 13, wherein said coating material is selected from the group consisting of: peptides, protein antigens, sugars and lipids.

15. Method for preparing a biomaterial, according to claim 13, wherein said coating material is extracellular matrix proteins from eukaryote cells or antibodies.

16. Biomaterial for suturing, according to claims 1-10, for its use in therapy.

17. Biomaterial for suturing, according to claims 1-10, for its use for bringing the edges of the tissue in suturing of accidental or surgical wounds together and for tissue repairing.

18. Biomaterial for suturing, according to claims 1-10, for its use in hemostasis, organ transplants, surgery of the gastrointestinal track, surgery of the urogenital tract, surgery of the respiratory tract, eye surgery, vascular surgery, plastic and reconstructive surgery, surgery on muscle tissue, epithelial tissue, nerve tissue and the repair of tendons, osseous tissue and cartilaginous tissue.

19. Use of the biomaterial for suturing, according to any one of claims 1-10, in the manufacture of material useful for bringing the edges of tissue together in suturing of accidental or surgical wounds and for tissue repairing.

20. Use of the biomaterial for suturing, according to any one of claims 1-10, in the manufacture of material useful for bringing the edges of tissue together in intestinal anastomosis.

21. Use of the biomaterial for suturing, according to any one of claims 1-10, in the manufacture of material useful for the attachment of prostheses.

22. Use of adult multipotent stem cell population derived from adipose tissue with proliferative and/or differentiating capacity as coating cellular population of a threads for suturing.

**23.** Use according to claim 22, **characterised in that** the adipose derived adult multipotent stem cellular population is an autologous, allogeneic or xenogeneic cellular population or a combination of these.

**Patentansprüche**

**1.** Biomaterial zum Naehen umfassend:

a) resorbierbare oder nicht-resorbierbare Fäden
b) aus Fettgewebe Erwachsener stammende multipotente Stammzellpopulation mit proliferativer und/oder

Differenzierungsfähigkeit, die die Fäden beschichtet.

**2.** Biomaterial gemäß Anspruch 1, wobei die Fäden natürlichen oder synthetischen Ursprungs sind.

**3.** Biomaterial gemäß den Ansprüchen 1 bis 2, wobei die Fäden eine Monofilament- oder Multifilament-Struktur aufweisen.

**4.** Biomaterial gemäß irgendeinem der vorhergehenden Ansprüche, wobei der Faden ein Nähfaden ist, der mit einer Nadel verbunden ist.

**5.** Biomaterial gemäß irgendeinem der vorhergehenden Ansprüche, wobei die aus Fettgewebe Erwachsener stammende multipotente Stammzellpopulation des Trägermaterials eine heterogene Zellpopulation ist.

**6.** Biomaterial gemäß irgendeinem der vorhergehenden Ansprüche, wobei die aus Fettgewebe Erwachsener stammende multipotente Stammzellpopulation der Beschichtung für das Trägermaterial genetisch modifizierte Zellen enthält.

**7.** Biomaterial gemäß Anspruch 6, wobei die aus Fettgewebe Erwachsener stammende multipotente Stammzellpopulation des Trägermaterials modifizierte Zellen enthält mit einem Gen, das ein Protein kodiert, welches bei der Geweberegeneration beteiligt ist.

**8.** Biomaterial gemäß irgendeinem der vorhergehenden Ansprüche, wobei die aus Fettgewebe Erwachsener stammende multipotente Stammzellpopulation für das Trägermaterial eine autologe, allogene oder xenogene Zellpopulation oder Kombinationen davon enthält.

**9.** Biomaterial gemäß Anspruch 8, wobei die aus Fettgewebe Erwachsener stammende multipotente Stammzellpopulation des Trägermaterials eine autologe Zellpopulation enthält.

**10.** Biomaterial gemäß Anspruch 8, wobei die aus Fettgewebe Erwachsener stammende multipotente Stammzellpopulation des Trägermaterials eine allogene Zellpopulation enthält.

**11.** Verfahren zur Herstellung eines Biomaterials gemäß irgendeinem der vorhergehenden Ansprüche, umfassend:

a) Vermehren der aus Fettgewebe Erwachsener stammenden multipotenten Stammzellpopulation,
b) Eintauchens des Nahtmaterials in ein geeignetes Kulturmedium für diese Zellpopulation,
c) Beimpfen einer Suspension der zuvor kultivierten Zellpopulation auf das Nahtmaterial,
d) Kultivieren der vorherigen Zubereitung unter den geeigneten Bedingungen,
e) Isolieren des Nahtmaterials mit der geeigneten zellulären Beschichtung.

**12.** Verfahren zur Herstellung eines Biomaterials zum Naehen gemäß dem vorhergehenden Anspruch, wobei die aus Fettgewebe Erwachsener stammende multipotente Stammzellpopulation durch Kleben mit dem Trägermaterial verbunden wird.

**13.** Verfahren zur Herstellung eines Biomaterials gemäß Anspruch 11 weiterhin umfassend eine vorherige Beschichtung der Fäden, die die Haftung der Zellpopulation ermöglicht.

**14.** Verfahren zur Herstellung eines Biomaterials gemäß Anspruch 13, wobei das Beschichtungsmaterial ausgewählt ist aus der Gruppe bestehend aus:

Peptiden, Proteinantigenen, Zuckern und Lipiden.

**15.** Verfahren zur Herstellung eines Biomaterials gemäß Anspruch 13, wobei das Beschichtungsmaterial extrazelluläre Matrixproteine aus eukaryotischen Zellen oder Antikörper darstellt.

**16.** Biomaterial zum Naehen gemäß den Ansprüchen 1 bis 10 zur Verwendung in der Therapie.

**17.** Biomaterial zum Naehen gemäß den Ansprüchen 1 bis 10 zur Verwendung beim Zusammenbringen der Ränder des Gewebes während des Nähens von unfallbedingten oder chirurgischen Wunden und zur Geweberegeneration.

**18.** Biomaterial zum Naehen gemäß den Ansprüchen 1 bis 10 zur Verwendung bei der Hämostase, Organtransplantationen, Operation des Magen-Darm-Traktes, Operation des Urogenitaltraktes, Operation der Atemwege, Augenchirurgie, Gefäßchirurgie, plastischen und rekonstruktiven Chirurgie, Operation von Muskelgewebe, Epithelgewebe, Nervengewebe und bei der Reparatur von Sehnen, Knochengewebe und Knorpelgewebe.

**19.** Verwendung des Biomaterials zum Naehen gemäß irgendeinem der Ansprüche 1 bis 10 zur Herstellung eines Materials, welches geeignet ist, die Ränder von Gewebe während des Nähens von unfallbedingten oder chirurgischen Wunden zusammenzubringen und welches zur Geweberegeneration geeignet ist.

**20.** Verwendung des Biomaterials zum Naehen gemäß irgendeinem der Ansprüche 1 bis 10 zur Herstellung eines Materials, welches geeignet ist, die Ränder von Gewebe bei Darmanastomose zusammenzubringen.

**21.** Verwendung des Biomaterials zum Naehen, gemäß irgendeinem der Ansprüche 1 bis 10 zur Herstellung eines Materials, das zur Anbringung von Prothesen geeignet ist.

**22.** Verwendung einer aus Fettgewebe Erwachsener stammenden multipotenten Stammzellpopulation mit proliferativer und/oder Differenzierungsfähigkeit als Zellpopulation zur Beschichtung von Fäden zum Naehen.

**23.** Verwendung gemäß Anspruch 22, **dadurch gekennzeichnet, dass** die aus Fettgewebe Erwachsener stammende multipotente Stammzellpopulation eine autologe, allogene oder xenogene Zellpopulation oder Kombinationen davon ist.

**Revendications**

**1.** Biomatériau pour sutures comprenant :

a) des fils résorbables ou non résorbables
b) une population de cellules souches adultes multipotentes issues du tissu adipeux ayant une capacité de prolifération et/ou de différenciation revêtant lesdits fils.

**2.** Biomatériau, selon la revendication 1, dans lequel les fils sont d'origine naturelle ou synthétique.

**3.** Biomatériau, selon les revendications 1 et 2, dans lequel les fils présentent une structure monofilament ou multifilament.

**4.** Biomatériau, selon l'une quelconque des revendications précédentes, dans lequel le fil est un fil de suture relié à une aiguille.

**5.** Biomatériau, selon l'une quelconque des revendications précédentes, dans lequel la population de cellules souches adultes multipotentes issues du tissu adipeux du matériau support est une population cellulaire hétérogène.

**6.** Biomatériau, selon l'une quelconque des revendications précédentes, dans lequel la population de cellules souches adultes multipotentes issues du tissu adipeux du revêtement pour le matériau support comprend des cellules génétiquement modifiées.

7. Biomatériau, selon la revendication 6, dans lequel la population de cellules souches adultes multipotentes issues du tissu adipeux du matériau support comprend des cellules modifiées avec un gène codant pour une protéine impliquée dans la réparation tissulaire.

8. Biomatériau, selon l'une quelconque des revendications précédentes, dans lequel la population de cellules souches adultes multipotentes issues du tissu adipeux pour le matériau support comprend une population cellulaire autologue, allogénique ou xénogénique ou une combinaison de celles-ci.

9. Biomatériau, selon la revendication 8, dans lequel la population de cellules souches adultes multipotentes issues du tissu adipeux du matériau support comprend une population cellulaire autologue.

10. Biomatériau, selon la revendication 8, dans lequel la population de cellules souches adultes multipotentes issues du tissu adipeux du matériau support comprend une population cellulaire allogénique.

11. Méthode de préparation d'un biomatériau, selon l'une quelconque des revendications précédentes, comprenant

a) l'expansion de la population de cellules souches adultes multipotentes issues du tissu adipeux,
b) l'immersion du matériau de suture dans un milieu de culture adapté à cette population cellulaire,
c) l'inoculation d'une suspension de la population cellulaire précédemment cultivée sur le matériau de suture,
d) la culture de la préparation précédente dans les conditions appropriées,
e) l'isolation du matériau de suture avec le revêtement cellulaire approprié.

12. Méthode de préparation d'un biomatériau pour sutures selon la revendication précédente, dans laquelle la population de cellules souches adultes multipotentes issues du tissu adipeux est reliée au matériau support par adhésion.

13. Méthode de préparation d'un biomatériau, selon la revendication 11, comprenant en outre un revêtement préalable des fils qui facilite l'adhérence de la population cellulaire.

14. Méthode de préparation d'un biomatériau, selon la revendication 13, dans laquelle ledit matériau de revêtement est sélectionné parmi le groupe constitué de : peptides, antigènes protéiques, sucres et lipides.

15. Méthode de préparation d'un biomatériau, selon la revendication 13, dans laquelle ledit matériau de revêtement est constitué de protéines de la matrice extracellulaire provenant de cellules eucaryotes ou d'anticorps.

16. Biomatériau pour sutures, selon les revendications 1 à 10, pour son utilisation dans la thérapie.

17. Biomatériau pour sutures, selon les revendications 1 à 10, pour son utilisation pour rapprocher des bords du tissu lors de la suture de plaies accidentelles ou chirurgicales et pour réparer des tissus.

18. Biomatériau pour sutures, selon les revendications 1 à 10, pour son utilisation dans l'hémostase, les greffes d'organes, la chirurgie de l'appareil gastro-intestinal, la chirurgie de l'appareil urogénital, la chirurgie de l'appareil respiratoire, la chirurgie oculaire, la chirurgie vasculaire, la chirurgie plastique et reconstructrice, la chirurgie sur tissu musculaire, tissu épithélial, tissu nerveux et la réparation des tendons, tissu osseux et tissu cartilagineux.

19. Utilisation du biomatériau pour sutures, selon l'une quelconque des revendications 1 à 10, dans la fabrication de matériau utile pour rapprocher les bords de tissu lors de la suture de plaies accidentelles ou chirurgicales et pour réparer des tissus.

20. Utilisation du biomatériau pour sutures, selon l'une quelconque des revendications 1 à 10, dans la fabrication de matériau utile pour rapprocher les bords de tissu lors d'une anastomose intestinale.

21. Utilisation du biomatériau pour sutures, selon l'une quelconque des revendications 1 à 10, dans la fabrication de matériau utile pour fixer des prothèses.

22. Utilisation d'une population de cellules souches adultes multipotentes issues du tissu adipeux ayant une capacité de prolifération et/ou de différenciation comme une population cellulaire de revêtement de fils de suture.

23. Utilisation selon la revendication 22, **caractérisée en ce que** la population de cellules souches adultes multipotentes issues du tissu adipeux consiste en une population cellulaire autologue, allogénique ou xénogénique ou une combinaison de celles-ci.

# FIGURE 1

# FIGURE 2

# FIGURE 3a

# FIGURE 3b

## FIGURE 4a

7 mm

## FIGURE 4b

7 mm

# FIGURE 5

EP 1 803 472 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 577047 A
- GB 1401842 A
- GB 1430554 A
- RU 2125469
- EP 0652017 A
- US 6264675 B

### Non-patent literature cited in the description

- **YOUNG et al.** *The Journal of Bone and Joint Surgery,* 1998, vol. 16, 406-13
- **GIMBLE et al.** *Expert Opinion on Biological Therapy,* 2003, vol. 3, 705-13
- **ARONSON SB ; MCMASTER RP ; MOORE TE JR ; COON MA.** Toxicity of the cyanoacrylates. *Arch Opthalmol,* September 1970, vol. 8 (3), 342-9
- **ASENCIO ARANA F ; MARTÍNEZ SORIANO F ; FENOLLOSA VAZQUEZ R.** Aproximación a los estudios de los anastomosis intestinales experimentales. Métodos bioquímicos, físicos y microangiográficos. *Cir Esp,* 1989, vol. 46, 805-810
- **BERESFORD JN ; BENNETT JH ; DEVLIN C ; LEBOY PS ; OWEN ME.** Evidence for an inverse relationship between the differentiation of adipocytic and osteogenic cells in rat marrow stromal cell cultures. *J. Cell Sci.,* June 1992, vol. 102, 341-51
- **CAPLAN AL.** Mesenchymal stem cells. *J Orthop Res,* September 1991, vol. 9 (5), 641-50
- **DE UGARTE DA ; MORIZONO K ; ELBARBARY A ; ALFONSO Z ; ZUK PA ; ZHU M ; DRAGOO JL ; ASHJIAN P ; THOMAS B ; BENHAIM P.** Comparison of multi-lineage cells from human adipose tissue and bone marrow. *Cells Tissues Organs,* 2003, vol. 174 (3), 101-9
- **ELLIS, H.** the aetiology of postoperative abdominal adhesions. An experimental study. *Br J Surg,* 1962, vol. 50, 10-16
- **FRIEDENSTEIN AJ.** Precursor cells of mechanocytes. *Int Rev Cytol.,* 1976, vol. 47, 327-59
- **HAYNESWORTH SE ; GOSHIMA J ; GOLBERG VM ; CAPLAN AL.** Characterization of cells with osteogenic potential from human marrow. *Bone,* 1992, vol. 13 (1), 81-8
- **JOHNSTONE B ; HERING TM ; CAPLAN AL ; GOLDBERG VM ; YOO JU.** In vitro chondrogenesis of bone marrow-derived mesenchymal progenitor cells. *Exp Cell Res,* 10 January 1998, vol. 238 (1), 265-72
- **MILDE LN.** An anaphylactic reaction to fibrin glue. *Anesth Analg,* November 1989, vol. 69 (5), 684-6
- **PITTENGER MF ; MACKAY AM ; BECK SC ; JAISWAL RK ; DOUGLAS R ; MOSCA JD ; MOORMAN MA ; SIMONETTE DW ; CRAIG S ; MARSHAK DR.** Multilineage potential of adult human mesenchymal stem cells. *Science,* 02 April 1999, vol. 284 (5411), 143-7
- **RAVO B.** Colorectal anastomotic healing and introcolonoic bypass procedure. *Surg Clin North Am,* 1988, vol. 68, 1267-1294
- **TSE WT ; PENDLETON JD ; BEYER WM ; EGALKA MC ; GUINAN EC.** Suppression of allogeneic T-cell proliferation by human marrow stromal cells: implications in transplantation. *Transplantation,* 15 February 2003, vol. 75 (3), 389-97
- **VERREET RP ; FAKIR C ; OHMANN C ; ROER HD.** Preventing recurrent postoperative adhesions: An experimental study in rats. *Eur Sug Res,* 1992, vol. 21, 267-273
- **WAKITANI S ; SAITO T ; CAPLAN AL.** Myogenic cells derived from rat bone marrow mesenchymal stem cells exposed to 5-azacytidine. *Muscle Nerve,* December 1995, vol. 18 (12), 1417-26
- **ZARAPICO ROMERO M ; SAEZ LÓPEZ DE RUEDA F.** La asociación fibrinodesoxirribonucleasa en la profilaxis de la adhesiones peritoneales post-operativas. *Rev Fac Med Sevilla,* 1972, vol. 20, 347-362
- **ZUK PA ; ZHU M ; ASHJIAN P ; DE UGARTE DA ; HUANG JI ; MIZUNO H ; ALFONSO ZC ; FRASER JK ; BENHAIM P ; HEDRICK MH.** Human adipose tissue is a source of multipotent stem cells. *Mol Biol Cell,* December 2002, vol. 13 (12), 4279-95
- **ZUK PA ; ZHU M ; MIZUNO H ; HUANG J ; FUTRELL JW ; KATZ AJ ; BENHAIM P ; LORENZ HP ; HEDRICK MH.** Multilineage cells from human adipose tissue: implications for cell-based therapies. *Tissue Eng,* April 2001, vol. 7 (2), 211-28